# EUROPEAN PATENT APPLICATION

(11) **EP 1 980 570 A2**
(43) Date of publication of application: **15.10.2008**
(21) Application number: 08155597.1
(22) Date of filing: 02.06.1999
(51) Int. Cl.: C07K 1/26, C07K 1/28

(54) **Purification of Antibodies**

(30) Priority: 02.06.1998 AU PP385598
(62) Divisional of application: 99955271.4
(71) Applicant: GRADIPORE LIMITED, North Ryde, NSW 2113 (AU)
(72) Inventor: Rylatt, Dennis Brian, New South Wales, 2122 (AU); Lim, Sharon, New South Wales 2010 (AU)
(74) Representative: Daniels, Jeffrey Nicholas

(57) **Abstract**

A method of separation of an antibody from a mixture of the antibody and at least one contaminant, the method comprising: a) placing the antibody and contaminant mixture in a first solvent stream, the first solvent stream being separated from a second solvent stream by an electrophoretic membrane; b) selecting the pH for the first solvent stream such that contaminants with an isoelectric point (pl) lower than the antibody to be separated will be charged; c) applying an electric potential between the two solvent streams causing movement of at least some of the contaminants through the membrane into the second solvent stream while the antibody is substantially retained in the first solvent stream, or if entering the membrane, being substantially prevented from entering the second solvent stream; d) optionally, periodically stopping and reversing the electric potential to cause movement of any antibody having entered the membrane to move back into the first solvent stream, wherein substantially not causing any contaminants that have entered the second solvent stream to re-enter first solvent stream; and e) repeating step c) and optionally step d) until the first solvent stream contains the desired purity of antibody.

## Description

### Technical Field

The present invention relates to methods suitable for purification of antibodies, particularly monoclonal antibodies from ascites fluid.

### Background Art

The processing of complex biological solutions is a major bottleneck in the biotechnology industry and there is a strong demand for cost effective technologies for the purification of naturally occurring and recombinant proteins. This is particularly true for antibodies including monoclonal antibodies, where there has been an on-going search for simple generic methods of purification. In particular, monoclonal antibodies have had an increasing number of research, therapeutic and diagnostic applications since their initial production in 1975. The difficulties in bioprocessing have meant that recoveries of monoclonal antibodies in existing purification schemes are rarely reported but are often in the range of 10 to 70%. Although new processes have become increasingly effective in terms of yield and recovery, commonly used processes often utilise harsh pH or ionic strength conditions for elution which may not always be compatible with maintaining maximal biological activity of antibodies.

The present inventor has found that preparative electrophoresis using Gradiflow technology (AU 601040) is particularly suitable for the purification of antibodies. In contrast to conventional methods, Gradiflow uses mild, non-denaturing buffers and conditions which may be utilised to produce more active antibody preparations.

### Disclosure of Invention

In a first aspect, the present invention consists in a method of separation of an antibody from a mixture of the antibody and at least one contaminant, the method comprising:
(a) placing the antibody and contaminant mixture in a first solvent stream, the first solvent stream being separated from a second solvent stream by an electrophoretic membrane;
(b) selecting a pH for the first solvent stream such that contaminants with an isoelectric point (p*I*) lower than the antibody to be separated will be charged;
(c) applying an electric potential between the two solvent streams causing movement of at least some of the contaminants through the membrane into the second solvent stream while the antibody is substantially retained in the first solvent stream, or if entering the membrane, being substantially prevented from entering the second solvent stream;
(d) optionally, periodically stopping and reversing the electric potential to cause movement of any antibody having entered the membrane to move back into the first solvent stream, wherein substantially not causing any contaminants that have entered the second solvent stream to re-enter first solvent stream; and
(e) repeating step (c) and optionally step (d) until the first solvent stream contains the desired purity of antibody.

Preferably, the antibody and contaminant mixture is a monoclonal antibody in ascitic fluid.

In a preferred embodiment of the first aspect of the present invention, the electrophoretic membrane has a molecular mass cut-off of about 50 to 150 kDa, preferably about 100 kDa. The p*I* of the antibody to be separated is usually obtained by isoelectric focusing (IEF). The pH of the first solvent stream is preferably about 7.5 to 9.5. Major protein contaminants, including albumin whose p*I* is well known to be 4.9, can be separated from the antibodies by being caused to transfer into the second solvent stream at pH 8.3.

In a second aspect, the present invention consists in a method of separation of an antibody from a mixture of the antibody and at least one contaminant, the method comprising:
(a) placing the separated antibody in a first solvent stream, the first solvent stream being separated from a second solvent stream by an electrophoretic membrane;
(b) selecting a pH of the first solvent stream such that the pH is within 1 pH unit of the p*I* of the antibody;
(c) applying an electric potential between the two solvent streams causing movement of at least some of the contaminants through the membrane into the second solvent stream while the antibody is substantially retained in the first solvent stream, or if entering the membrane, being substantially prevented from entering the second solvent stream;
(d) optionally, periodically stopping and reversing the electric potential to cause movement of any antibody having entered the membrane to move back into the first solvent stream, wherein substantially not causing any contaminants that have entered the second solvent stream to re-enter first solvent stream; and
(e) repeating step (c) and optionally step (d) until the first solvent stream contains the desired purity of antibody.

The electrophoretic membrane in step (a) preferably has a molecular mass cut-off at least about 200 kDa to ensure the contaminants can pass through to the second solvent stream. A cartridge containing a large 1000 kDa pore size separating-membrane has been found to be particularly suitable for this aspect of the present invention. The pH in step (b) is typically from about 6 to 8.0. It will be appreciated that the pH of the buffer will depend on the p*I* of the antibody to be purified and the p*I*s of the contaminants.

The pH of the buffer used in step (b) can be above or below the p*I* of the antibody to be separated. Preferably, the pH is within 0.5 pH units of the p*I* of the antibody.

In a third aspect, the present invention consists in a method of separation of an antibody from a mixture of the antibody and at least one contaminant, the method comprising:
(a - e) separating the antibody according to the first aspect of the present invention;
(f) placing the separated antibody in a fresh first solvent stream, the first solvent stream being separated from a second solvent stream by an electrophoretic membrane;
(g) selecting a pH of the fresh first solvent stream such that the pH is within 1 pH unit of the p*I* of the antibody;
(h) applying an electric potential between the two solvent streams causing movement of at least some of the contaminants through the membrane into the second solvent stream while the antibody is substantially retained in the fresh first solvent stream, or if entering the membrane, being substantially prevented from entering the second solvent stream;
(i) optionally, periodically stopping and reversing the electric potential to cause movement of any antibody having entered the membrane to move back into the fresh first solvent stream, wherein substantially not causing any contaminants that have entered the second solvent stream to re-enter first solvent stream: and
(j) repeating step (h) and optionally step (i) until the fresh first solvent stream contains the desired purity of antibody.

The molecular mass cut-off of the electrophoretic membrane used in step (f) is preferably larger than the membrane used in step (b). The electrophoretic membrane in step (f) preferably has a molecular mass cut-off at least about 200 kDa to ensure the contaminants can pass through to the second solvent stream. A cartridge containing a large 1000 kDa pore size separating-membrane has been found to be particularly suitable for this aspect of the present invention. The pH in step (g) is typically from about 6 to 8.0. It will be appreciated that the pH of the buffer will depend on the p*I* of the antibody to be purified and the p*I*s of the contaminants.

The pH of the buffer used in step (g) can be above or below the p*I* of the antibody to be separated. Preferably, the pH is within 0.5 pH units of the p*I* of the antibody.

The present inventors have been able to obtain percent recoveries of monoclonal antibodies from ascitic fluid of at least 70% and often greater than 90% using the methods according to the present invention.

A significant advantage of the present invention is that the separated antibody is less denatured or altered compared with the same antibody obtained by conventional antibody purification methods such as salt precipitation, Protein-A, Protein-G and ion-exchange chromatography.

In a fourth aspect the present invention consists in use of Gradiflow in the purification and/or separation of antibodies.

In a fifth aspect, the present invention consists in an antibody purified by the method according to the first, second or third aspects of the present invention.

In order that the present invention may be more clearly understood, preferred forms will be described in the following examples with reference to the accompanying drawings.

### Brief Description of Drawings

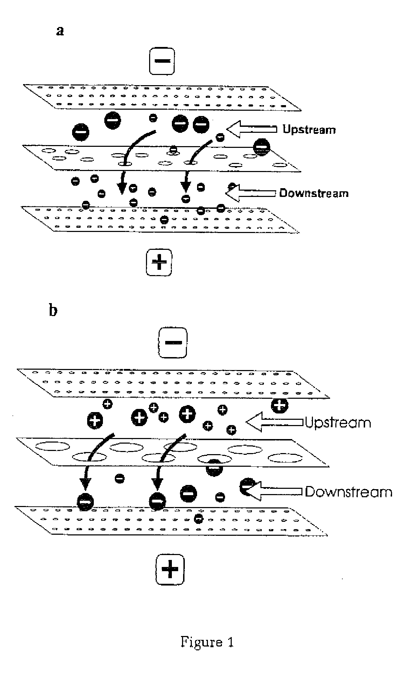

Figure 1 shows the operating modes of the Gradiflow separating cartridge. (a) Sized-based separation - this is a first step in which major contaminants are removed downstream from antibody mixture upstream. (b) Charge-based separation - this is a second step suitable to remove any residual contaminants if requiring antibodies of higher purity.

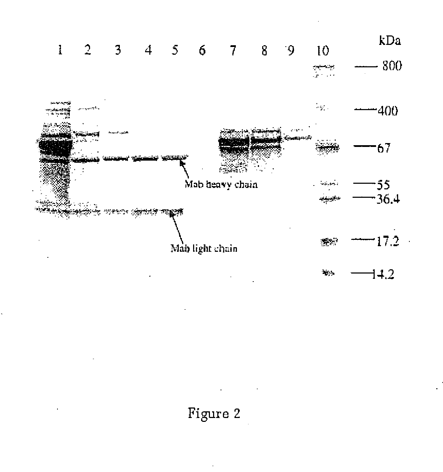

Figure 2 shows SDS-PAGE of the purification of antibody 4. Lane 1 is upstream at 0 min, lanes 2-5 upstream after 10, 20, 30, and 40 min, respectively. Lane 6-9 show downstream at 10, 20, 30, and 40 min, respectively. Lane 10 contains SDS molecular mass markers.
   Mab=monoclonal antibodies; kDa=Kilodaltons.

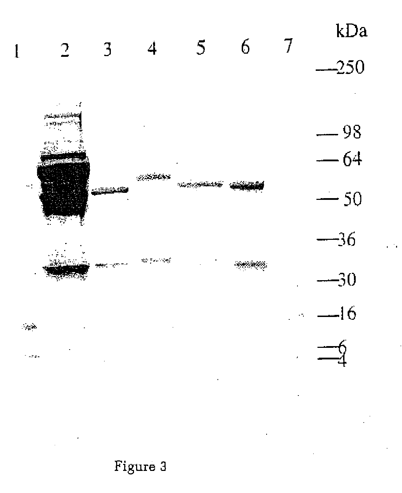

Figure 3 shows SDS-PAGE of the purified monoclonal antibodies according to the second aspect of the present invention. Lane 1 and 7 are the SDS-PAGE molecular weight markers. For contrast, the starting material for antibody 1 was placed in lane 2 while lanes 3, 4, 5 and 6 contain the final four product antibodies.

### Modes for Carrying Out the Invention

### EXPERIMENTAL

### Antibodies

The antibodies used in this study were all generated by conventional procedures (Bundesen et al 1985) and supplied to Gradipore Limited by Agen Biomedical Brisbane, Australia as murine ascitic fluids. Table I contains the properties of the target monoclonal antibodies.

**Table 1. Monoclonal antibodies (recovery as determined by EIA)**

| Antibody | Isotype | p*I* | Recovery | |
|---|---|---|---|---|
| | | | % | mg/ml antibody |
| 1 | IgG1 | pH 7.3-7.5 | 94^{a} | 8.9 |
| 2 | IgG2a | pH 6.7-7.7 | 73^{b} | 8.1 |
| 3 | IgG2b | pH 6.6-6.9 | 79^{b} | 10.8 |
| 4 | IgG1 | pH 6.8-7.0 | 71^{b} | 10.0 |

| | | | | |
|---|---|---|---|---|
| ^{a}- denotes one step purification ^{b} - denotes a two-step purification | | | | |

### Gradiflow technology

The separating cartridge of the Gradiflow contains a set of polyacrylamide-based restriction and separating membranes to enable the separation of macromolecules on the basis of size and/or charge (see Figure 1). A range of cartridges is available with *M*ᵣ cut-offs ranging from 25,000 to 1,000,000. The ability to fractionate proteins over a range of pH and the use of membranes of different pore sizes enables any target protein to be separated by virtue of its size or isoelectric point.

The Model LM1000 (Gradipore Limited, Sydney, Australia) contains peristaltic pumps, peltier coolers and power supply. It is controlled by a personal computer under a Windows 95 and Lab View format. Alternatively, a manually configured instrument is also available which can operate with conventional peristaltic pumps and power supply (Margolis et al 1995; Corthals et al 1996: Horvath et al 1996; Corthals et al 1997).

### Modes of separation

### Size-based separation (Figure 1a)

For size separation, a pH is selected at which all proteins have the same charge, in this case negative. Hence all of the proteins from the mixture circulating in the "upstream" compartment will try to migrate into the "downstream" compartment. If a membrane of restrictive pore size is selected, for example the Mᵣ 100,000 used in this case, molecules larger than Mᵣ 100,000 (such as the target antibodies) will be unable to transfer across the membrane and remain upstream. As essentially all proteins in mouse ascitic fluid have p*I* values less than pH 7.7, pH 8.3 was selected for size preparation in this paper. Under these conditions most of the ascitic proteins are transferred "downstream" leaving behind the M, 160 000 antibody molecules.

### Charged based separation (Figure 1b)

For charge-based separation, a pH is selected between the isoelectric points of two proteins such that one protein will have a positive charge and the other a negative charge. In this example, the protein mixture continuously circulates in the "upstream" compartment. When the current is applied, the negatively charged protein migrates through the membrane to the "downstream" compartment. Continuous circulation of the upstream and downstream compartments allows complete separation of the two proteins.

The vast majority of non-antibody proteins in murine ascitic fluid have isoelectric points below pH 6.5 so that at a pH above pH 6.5, these proteins are negatively charged and will migrate downstream leaving behind the antibody which normally has a p*I* above pH 6.6. For charge separations, a membrane with a large pore size is usually employed (*M*ᵣ 1 x 10⁶) to allow for maximum transport across the membrane.

### Purification of monoclonal antibodies

Each sample of ascitic fluid (0.5 - 2 ml) was diluted with at least three volumes of a buffer containing 40 mM Tris-borate, 1 mM EDTA pH 8.3. Firstly a size separation of each sample was carried out in this buffer for 30 to 40 min at 200 V with a Mᵣ cut-off 100,000 separating membrane. Under these conditions, albumin and other impurities rapidly migrated across the membrane leaving behind the purified antibody upstream.

For higher purity, a second run was selected at a pH close to the p*I* of each specific antibody using a Mᵣ cut-off 1 x 10⁶ membrane. For example 40 mM Tris buffer can be adjusted to the required pH with acetic acid. The run time was 40 min at 200 V. The remaining impurities migrated through the membrane while the antibody remained upstream. By taking 50 µl aliquots of upstream and downstream at 10-min intervals during the first and second run, the purity of the target antibodies was then determined by denaturing sodium dodecyl sulfate-polyacrylamide gel electrophoresis (SDS-PAGE). The percent recovery was determined by enzyme immunoassay (EIA) after the runs were completed.

The upstream and downstream were harvested after 40 min. For maximum recovery of antibody, a small amount (7 ml) of running buffer was pumped in the upstream and downstream for a minute at the end of the separation process with the current reversed. After the current reversal was switched off, the upstream and downstream were allowed to circulate for another minute before the upstream wash was harvested and combined with the initial antibody harvested. An additional 10-15% of antibody can be recovered in this washing process

### Isoelectric focusing

The p*I* of each antibody was determined by running an isoelectric focusing (IEF) gel using a Novex (San Diego CA, USA) IEF gel apparatus as described by the manufacturer. Briefly, the running conditions involved a run time of 1 h at 100 V 1 h at 200 V and 500 V for 30 min. The IEF gel was fixed with a solution of 12% (w/v) trichloroacetic acid (Sigma Product No T8657) with 3.5% (w/v) 5-sulfosalicylic acid (Sigma Product No S-3147) in deionised water for 30 min before staining with Gradipure^{™} Coomassie Blue.

### Determination of antibody purity

Samples were analysed by SDS-PAGE on Gradipore 4-20% T SDS gels [T=(g acrylamide + g *N,N*-methylenebisacrylamide)/100 ml solution]. The changes to sample purity with time were determined by comparing the protein bands upstream and downstream at different times.

### Determination of antibody recovery

### Protein concentration

Protein levels in the upstream and downstream were determined by measuring the ultraviolet absorption at 280 nm. A 1 mg/ml solution of mouse monoclonal antibodies was assumed to have an absorbance of 1.2 AU.

### Enzyme immunoassay

The antibody activity was determined by a two-site EIA using either antigen or unlabelled sheep anti-mouse immunoglobulin as the capturing component. The microplate was first coated with either 50 µl of antigen (10 µl/ml) or rabbit anti-mouse immunoglobulins (10 µl/ml) Dako (Carpinteria, USA) in phosphate-buffered saline (PBS) pH 7.4 for 1 h at room temperature.

Excess antigen was removed by inverting and tapping the plate and the plate was washed three times with PBS containing 0.1% Tween 20 (PBS/T). Next, 50 µl of a suitable dilution in PBS/T of the monoclonal fraction under test was added and the incubation was allowed to proceed for an hour at room temperature. After removal of unbound antibody by washing, bound antibody was labelled by the addition of a 1/1000 dilution of horseradish peroxidase (HRPO)-labelled anti anti-mouse antibody and incubated for another hour. Finally, the bound enzyme detected after further washing by the addition of substrate and stop solutions.

### RESULTS AND DISCUSSION

### Isoelectric focusing

IEF is a technique that enables proteins to be characterised by their p*I* values which can be used to determine the best conditions for a charge based separation. An IEF gel of the starting material showed that each ascitic fluid had a unique IEF pattern with the major difference being the position of the multiple bands of target antibodies.

The IEF gel indicated a range of p*I* values from 6.6 to 7.7 for the four different antibodies derived from ascitic fluid samples. The p*I* values of the antibodies are listed in Table 1. The variety of isoforms provides plausible reason for the low recoveries from conventional ion-exchange protocols for antibodies as the charge heterogeneity could cause multiple broad peaks and tailing effects.

### Purification of mouse antibodies

### Size separation

Size exclusion was chosen as the first step when IEF (Figure 1) revealed a wide variation in the isoelectric charge of individual antibodies. A membrane with a *M*ᵣ cut-off of 100,000 was selected as this pore size should retain the *M*ᵣ 160,000 antibody, yet allow the rapid passage of smaller protein molecules. A pH of 8.3 was chosen so that the majority of immune ascitic fluid proteins had net negative charges at this pH.

A time course for the purification of antibody 4 is depicted in Figure 2. Similar results were obtained for the other three antibodies. After 20 min, the most significant bands in the sample stream are the characteristic heavy and light chains of the monoclonal antibody. Acceptable purity was achieved after 30 min (lane 5 in Figure 2) without substantial improvement at 40 min. Lower-molecular-mass proteins with the most abundant being mouse serum albumin, rapidly passed through the *M*ᵣ 100,000 membrane leaving behind the antibody upstream. The downstream was harvested every 10 min and showed decreasing amounts of protein in each subsequent harvest (Figure 2, lanes 7-9). Most of the impurities were removed from the antibody in the first 10 min (lane 7) with large amounts of albumin present in the two initial downstream harvests that were collected at 10 min and 20 min. Albumin had disappeared from the upstream after 10 min. This ending was interpreted, as residual albumin found downstream in the 10-min sample being in transit within the separation membrane when the 10-min sample was taken.

These conditions were chosen for initial separation with a view to try to select a universal first step that would allow the purification of any antibody in good yield with the high degree of purity. The Tris-borate buffer was selected because it has proven to be a useful butter for separations under native conditions in many other applications. The voltage was selected on the basis of the required speed of the separation with the aim of completing the process in less than 30 min. For most applications, the higher the voltage the more rapid the purification.

Some antibodies are thought to be labile at room temperature and there are many reports that temperature can affect protein folding with lower temperatures increasing protein volubility.

### Charge separation

A higher degree of purity for each antibody can be achieved in a charged-based second step carried out at a pH near the p*I* of each antibody. As the isoelectric points vary, this meant a different pH was selected for each antibody. During the separations using either size or charge, there was no evidence that the solubility of each target antibody was affected by a pH higher than its p*I* or near its p*I*. The solubility of the proteins remained excellent with these operating conditions and choice of buffers used. Each different antibody solution was adjusted with acetic acid to the pH that was close to the p*I* prior to a charge separation. The membrane used for the charge separation was a *M*ᵣ 1 x 10⁶ pore size to increase the speed of removal of the contaminating proteins. Under these conditions, the monoclonal antibody now uncharged, remained upstream and the contaminating negatively charged proteins migrated downstream SDS-PAGE (Figure 3) indicated that a high degree of purity was achieved for all antibodies using this second step. The relatively high p*I* of antibody 1 allowed this antibody to be purified in a single step at pH 8.3 using Mᵣ 1 x 10⁶ pore size membrane (instead of *M*ᵣ 100,000). This antibody was expected to migrate through the Mᵣ 1 x 10⁶ membrane because it did have a small negative charge at this pH but for short runs of less than an hour, it remained upstream. Indeed with longer separation times (1-2 h) some migration downstream was noted. Recovery

The recovery of the monoclonal antibodies was determined by comparing the final biological activity of the antibody present upstream, downstream and in the washes with the starting material (Table I). Although recoveries for antibodies 2, 3 and 4 (using two-stage purifications) were less than for antibody 1 (Table 1) quantities of >8 mg of each antibody were recovered per ml of ascitic fluid. This recovery was higher than the expected yields for other purification procedures. These higher activities recovered may be due to a combination of the mild buffers used the relatively short distance required for separation to occur with the separation cartridge and the short purification times.

The protocol developed takes the advantage of the large size (*M*ᵣ 160,000) of the monoclonal antibodies relative to other proteins in ascitic fluid and their relatively high p*I* values compared to other proteins in murine ascitic fluid. The protocol described here is less complex than conventional chromatographic purification techniques where more variables need to be considered. The immunoglobulin fraction is usually about 2-25% of the total protein in ascitic fluids. The high content of lipid present in ascitic fluid is know to reduce the life of fractionation columns. In the present experiments, excellent purification and recovery without the need to delipidise or pretreat the samples was achieved.

Initially, small amounts (10-20 mg) of each target antibody were purified to test the invention. Linear scale up from the 15 cm² membranes (used here) to 200 cm² has been accomplished by the present inventor and co-workers previously for haemoglobin/albumin separations (Horvath et al 1994). Preliminary scale up from 2 ml to 10 ml of mouse ascites fluid on the 15 cm² cartridges was achieved. Longer processing time or a larger separation cartridges may be required for larger quantities of proteins.

Comparison of antibodies separated according to the present invention with the same antibodies purified by standard techniques including Protein-A, Protein-G and ion exchange chromatography by a collaborating third party revealed the antibodies obtained by the present invention were more active. Furthermore, the yields of antibodies were greater using the present invention. Tables 2 and 3 show the results of the comparisons of several purifications of antibodies.

**Table 2. Yield of Mouse IgG1 monoclonal antibody**

| Purification Method | Yield (mg antibody recovered) |
|---|---|
| Protein-A | 65 |
| Invention | 68 |
| Ion exchange | 60 |

**Table 3. Yield of Rat IgG2b monoclonal antibody**

| Purification Method | Yield (%) |
|---|---|
| Protein-G | 83 |
| Invention | 91 |
| Ion exchange | 81 |

Electrophoretic analysis (SDS-PAGE) of antibodies purified by the present invention revealed lower number of protein bands indicating that the present method does not cleave or damage the antibodies compared with other purification techniques. Not only can the present invention provide greater yields of antibody, the invention also provides more active and less denatured antibody preparations.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### REFERENCES

PG Bundesen, DM Wyatt, LE Cottis, AS Blake, DA Massingham, WA Fletcher, G Street, JS Welch, DB Rylatt Vet Immunol Immunopath 8 (1985) 245-260.
Horvath ZC, Corthals GL, Wrigley CW and Margolis J. Multifunctional apparatus for electrokinetic processing of proteins. Electrophoresis 1994 15 968-971.
Margolis J, Corthals G and Horvath ZS. Preparative reflux electrophoresis. Electrophoresis 1995 16 98-100.
Corthals GL, Margolis J, Williams KL and Gooley AA. The role of pH and membrane porosity in preparative electrophoresis. Electrophoresis 1996 17 771-775.
Horvath ZC, Gooley AA, Wrigley CW, Margolis J and Williams KL. Preparative affinity membrane electrophoresis. Electrophoresis 1996 17 224-226.
Corthals GL, Molloy MP, Herbert BR, Williams KL and Gooley AA. Prefractionation of protein samples prior to two-dimensional electrophoresis. Electrophoresis 1997 18 317-323.

## Claims

1. A method of separating an antibody from a mixture of antibody and at least one contaminant, the method comprising:
(a) placing the antibody and contaminant mixture in a first solvent stream, the first solvent stream being separated from a second solvent stream by an electrophoretic membrane having a molecular mass cut-off of 50 to 150 kDa;
(b) selecting a pH of 7.5 to 9.5 for the first solvent stream such that contaminants with an isoelectric point (p*I*) lower than the antibody to be separated will be charged:
(c) applying an electric potential between the two solvent streams causing movement of at least some of the contaminants through the membrane into the second solvent stream while the antibody is substantially retained in the first solvent stream, or if entering the membrane, being substantially prevented from entering the second solvent stream:
(d) optionally, periodically stopping and reversing the electric potential to cause movement of any antibody having entered the membrane to move back into the first solvent stream, wherein substantially not causing any contaminants that have entered the second solvent stream to re-enter the first solvent stream: and
(e) repeating step (c) and optionally step (d) until the first solvent stream contains the desired purity of antibody, wherein the recovery of the antibody is at least 70% and the antibody being less denatured or altered compared with the same antibody obtained by conventional antibody purification methods.

2. The method according to claim 1 wherein the antibody and contaminant mixture is a monoclonal antibody in ascitic fluid.

3. The method according to claim 1 or 2 wherein the electrophoretic membrane has a molecular mass cut-off of 100 kDa.

4. The method according to any one of claims 1 to 3 further including the steps of:
(f) placing the separated antibody in a fresh first solvent stream, the fresh first solvent stream being separated from a second solvent stream by an electrophoretic membrane having a molecular mass cut-off larger than that of the electrophoretic membrane used in step (b);
(g) selecting a pH of the fresh first solvent stream such that the pH is within 1 pH unit of the p*I* of the antibody;
(h) applying an electric potential between the two solvent streams causing movement of at least some of the contaminants through the membrane into the second solvent stream while the antibody is substantially retained in the fresh first solvent stream, or if entering the membrane, being substantially prevented from entering the second solvent stream:
(i) optionally, periodically stopping and reversing the electric potential to cause movement of any antibody having entered the membrane to move back into the fresh first solvent stream, wherein substantially not causing any contaminants that have entered the second solvent stream to re-enter the fresh first solvent stream: and
(j) repeating step (h) and optionally step (i) until the fresh first solvent stream contains the desired purity of antibody, wherein the recovery of the antibody is at least 70%.

5. The method according to claim 4 wherein the molecular mass cut-off of the electrophoretic membrane used in step (f) is at least 200 kDa.

6. The method according to claim 4 wherein the molecular mass cut-off of the electrophoretic membrane used in step (f) is 1000 kDa.

7. The method according to any one of claims 4 to 6 wherein the pH of the fresh first solvent stream in step (g) is from 6 to 8.

8. The method according any one of claims 4 to 6 wherein the pH of the fresh first solvent stream in step (g) is within 0.5 pH units of the p*I* of the antibody.

9. The method according to any one of claims 1 to 8 wherein recovery of the antibody is at least 90%.

10. A method of separating an antibody from a mixture of antibody and at least one contaminant, the method comprising:
(a) placing the antibody and contaminant mixture in a first solvent stream, the first solvent stream being separated from a second solvent stream by an electrophoretic membrane having a molecular mass cut-off of at least 200 kDa:
(b) selecting a pH of the first solvent stream such that the pH is within 1 pH unit of the p*I* of the antibody;
(c) applying an electric potential between the two solvent streams causing movement of at least some of the contaminants through the membrane into the second solvent stream while the antibody is substantially retained in the first solvent stream, or if entering the membrane, being substantially prevented from entering the second solvent stream:
(d) optionally, periodically stopping and reversing the electric potential to cause movement of any antibody having entered the membrane to move back into the first solvent stream, wherein substantially not causing any contaminants that have entered the second solvent stream to re-enter the first solvent stream; and
(e) repeating step (c) and optionally step (d) until the first solvent stream contains the desired purity of antibody, wherein the recovery of the antibody is at least 70% and the antibody being less denatured or altered compared with the same antibody obtained by conventional antibody purification methods.

11. The method according to claim 10 wherein the antibody and contaminant mixture is a monoclonal antibody in ascitic fluid.

12. The method according to claim 10 or 11 wherein the molecular mass cut-off of the electrophoretic membrane used in step (a) is 1000 kDa.

13. The method according to any one of claims 10 to 12 wherein the pH of the first solvent stream in step (b) is from 6 to 8.

14. The method according to any one of claims 10 to 12 wherein the pH of the first solvent stream in step (b) is within 0.5 pH units of the p*I* of the antibody.

15. The method according to any one of claims 10 to 14 wherein recovery of the antibody is at least 90%.
